(19) Europäisches Patentamt
European Patent Office
Office européen des brevets

(11) EP 4 702 978 A1

# (12) EUROPEAN PATENT APPLICATION
published in accordance with Art. 153(4) EPC

(43) Date of publication:
**04.03.2026 Bulletin 2026/10**

(21) Application number: **23934313.0**

(22) Date of filing: **27.04.2023**

(51) International Patent Classification (IPC):
***A61K 31/42*** *(2006.01)* ***A61P 25/28*** *(2006.01)*

(52) Cooperative Patent Classification (CPC):
**A61K 31/42; A61P 25/28**

(86) International application number:
**PCT/JP2023/016569**

(87) International publication number:
**WO 2024/224537 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Socium Inc.**
**Tokyo, 103-0026 (JP)**

(72) Inventors:
- **HORIMOTO, Katsuhisa**
  **Tokyo 103-0026 (JP)**
- **KIBOKU, Takayuki**
  **Tokyo 103-0026 (JP)**

(74) Representative: **De Clercq & Partners**
**Edgard Gevaertdreef 10a**
**9830 Sint-Martens-Latem (BE)**

## (54) AGENT FOR TREATING OR PREVENTING AMYOTROPHIC LATERAL SCLEROSIS

(57) An agent for treating or preventing amyotrophic lateral sclerosis, wherein an active component of the agent essentially consists of at least one selected from the group consisting of cycloserine, terizidone, and salts thereof. The active component may consist of at least one selected from the group consisting of cycloserine, terizidone, and salts thereof. The cycloserine may be D-cycloserine. The cycloserine may be L-cycloserine.

**Fig. 1**

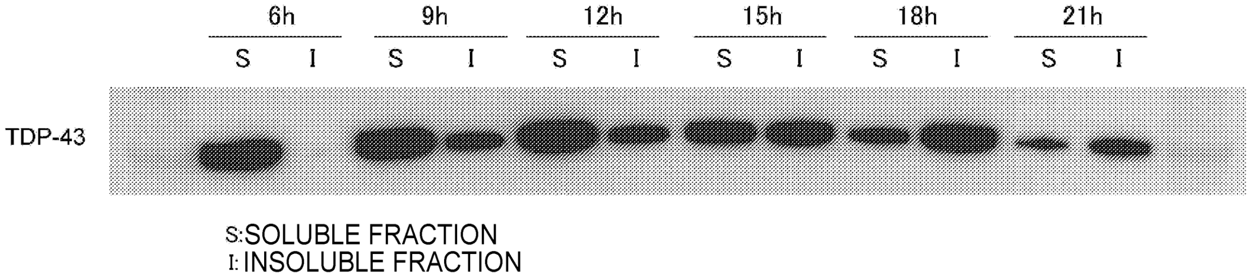


EP 4 702 978 A1

## Description

### Technical Field

The present invention relates to an agent for treating or preventing amyotrophic lateral sclerosis.

### Background Art

TDP-43 (TAR DNA-binding protein of 43kDa) is a type of heterogeneous nuclear ribonucleoprotein. TDP-43 has been identified as a major structural protein of ubiquitin-positive inclusions that appear in degenerated nerve cells and glial cells in amyotrophic lateral sclerosis (ALS) and frontotemporal lobar degeneration (FTLD). TDP-43 is a nuclear-localized protein, but in cells in which ubiquitin-positive inclusions are formed, TDP-43 translocates from the nucleus to the cytoplasm, aggregates, becomes insoluble, and accumulates in the cytoplasm.

TDP-43, which accumulates in the brains and spinal cords of ALS patients and FTLD patients, is abnormally phosphorylated at a plurality of serine residues at the C-terminal. In ALS patients and FTLD patients, it is not necessary for the full length of TDP-43 molecules to aggregate, rather, C-terminal fragments of TDP-43 aggregate. Previous studies have strongly suggested that TDP-43 aggregation causes abnormalities in RNA metabolism and induces cytotoxicity, leading to the onset and progression of various diseases.

As an example of diseases associated with TDP-43 aggregation, TDP-43 proteinopathy is known. Patent Literature 1 proposes the use of N,N,N',N'-tetramethyl-10H-phenothiazine-3,7-diaminium bis(methanesulfonate) as an agent for treating or preventing TDP-43 proteinopathy.

### Citation List

### Patent Literature

Patent Literature 1: Japanese Patent No. 5898701

Patent Literature 2: Japanese Translation of PCT Application No. 2022-500397

Patent Literature 3: Japanese Translation of PCT Application No. 2018-526345

Patent Literature 4: U.S. Patent Application Publication No. 2011/0160260

Patent Literature 5: Japanese Translation of PCT Application No. 2002-511409

### Summary of Invention

### Technical Problem

One object of the present invention is to provide an agent for treating or preventing amyotrophic lateral sclerosis.

### Solution to Problem

An aspect of the present invention provides an agent for treating or preventing amyotrophic lateral sclerosis, wherein an active component of the agent essentially consists of at least one selected from the group consisting of cycloserine, terizidone, and salts thereof.

In the agent for treating or preventing amyotrophic lateral sclerosis, the active component may consist of at least one selected from the group consisting of cycloserine, terizidone, and salts thereof.

In the agent for treating or preventing amyotrophic lateral sclerosis, the active component may essentially consist of cycloserine or salt thereof.

In the agent for treating or preventing amyotrophic lateral sclerosis, the active component may consist of cycloserine or salt thereof.

In the agent for treating or preventing amyotrophic lateral sclerosis, the cycloserine may be D-cycloserine.

In the agent for treating or preventing amyotrophic lateral sclerosis, the cycloserine may be L-cycloserine.

In the agent for treating or preventing amyotrophic lateral sclerosis, the agent may contain 15 mg or more and 100 mg or less of the active component.

In the agent for treating or preventing amyotrophic lateral sclerosis, the agent may contain 15 mg or more and 100 mg or less of D-cycloserine.

In the agent for treating or preventing amyotrophic lateral sclerosis, the active component may not be in a cationic form.

In the agent for treating or preventing amyotrophic lateral sclerosis, the active component may not be combined with a compound in an anionic form.

In the agent for treating or preventing amyotrophic lateral sclerosis, the active component may not be combined with acamprosate.

An aspect of the present invention provides a use of an active component essentially consisting of at least one selected from the group consisting of cycloserine, terizidone, and salts thereof, in manufacturing an agent for treating or preventing amyotrophic lateral sclerosis.

In the use, the active component may consist of at least one selected from the group consisting of cycloserine, terizidone, and salts thereof.

In the use, the active component may essentially consist of cycloserine or salt thereof.

In the use, the active component may consist of cycloserine or salt thereof.

In the use, the cycloserine may be D-cycloserine.

In the use, the cycloserine may be L-cycloserine.

In the use, the agent for treating or preventing amyotrophic lateral sclerosis may contain 15 mg or more and 100 mg or less of the active component.

In the use, the agent for treating or preventing amyotrophic lateral sclerosis may contain 15 mg or more and 100 mg or less of D-cycloserine.

In the use, the active component may not be in a cationic form.

In the use, the active component may not be combined with a compound in an anionic form.

In the use, the active component may not be combined with acamprosate.

An aspect of the present invention provides a method for treating or preventing amyotrophic lateral sclerosis, the method including administering an agent for treating or preventing amyotrophic lateral sclerosis to a patient with amyotrophic lateral sclerosis, wherein an active component of the agent essentially consists of at least one selected from the group consisting of cycloserine, terizidone, and salts thereof.

In the method of treating or preventing amyotrophic lateral sclerosis, the active component in the agent for treating or preventing amyotrophic lateral sclerosis may consist of at least one selected from the group consisting of cycloserine, terizidone, and salts thereof.

In the method of treating or preventing amyotrophic lateral sclerosis, the active component in the agent for treating or preventing amyotrophic lateral sclerosis may essentially consist of cycloserine or salt thereof.

In the method of treating or preventing amyotrophic lateral sclerosis, the active component in the agent for treating or preventing amyotrophic lateral sclerosis may consist of cycloserine or salt thereof.

In the method of treating or preventing amyotrophic lateral sclerosis, the cycloserine may be D-cycloserine. In the method of treating or preventing amyotrophic lateral sclerosis, the cycloserine may be L-cycloserine.

In the method of treating or preventing amyotrophic lateral sclerosis, the agent for treating or preventing amyotrophic lateral sclerosis may contain 15 mg or more and 100 mg or less of the active component.

In the method of treating or preventing amyotrophic lateral sclerosis, the agent for treating or preventing amyotrophic lateral sclerosis may contain 15 mg or more and 100 mg or less of D-cycloserine.

In the method of treating or preventing amyotrophic lateral sclerosis, the active component in the agent for treating or preventing amyotrophic lateral sclerosis may not be in a cationic form.

In the method of treating or preventing amyotrophic lateral sclerosis, the active component in the agent for treating or preventing amyotrophic lateral sclerosis may not be combined with a compound in an anionic form.

In the method of treating or preventing amyotrophic lateral sclerosis, the active component in the agent for treating or preventing amyotrophic lateral sclerosis may not be combined with acamprosate.

## Advantageous Effect of Invention

According to the present invention, it is possible to provide an agent for treating or preventing amyotrophic lateral sclerosis.

## Brief Description of Drawings

[Figure 1] Figure 1 is an image showing soluble fractions of TDP-43 and insoluble fractions of TDP-43 in Reference Example 1.

[Figure 2] Figure 2 is a graph showing the relationship between the elapsed time from transfection of the TDP-43 gene in Reference Example 1 and the proportion of insoluble fractions of TDP-43 in cells.

[Figure 3] Figure 3 shows images of cells in Example 1.

[Figure 4] Figure 4 is a graph showing the relationship between the concentration of a compound in Example 1 and the relative cell viability.

[Figure 5] Figure 5 is an image showing soluble fractions of TDP-43 and insoluble fractions of TDP-43 in Example 2.

[Figure 6] Figure 6 is a graph showing the relationship between a compound in Example 2 and the proportion of insoluble fractions of TDP-43 in cells.

[Figure 7] Figure 7 is an image showing soluble fractions of TDP-43 and insoluble fractions of TDP-43 in Example 3.

[Figure 8] Figure 8 is a graph showing the proportion of insoluble fractions of TDP-43 in Example 3.

## Description of Embodiments

Embodiments of the present invention will be described below. However, it should not be understood that the following embodiments limit the present invention. Those skilled in the art can clearly understand various alternative embodiments, examples and operational techniques from the present disclosure. It should be understood that the present invention includes various

embodiments and the like that are not described herein.

In an agent for treating or preventing amyotrophic lateral sclerosis (ALS) according to an embodiment, an active component essentially consists of at least one selected from among cycloserine and physiologically acceptable salts thereof. In the present disclosure, "essentially consists of" means that the active component does not include other components, but the presence of components other than the active component, such as excipients and/or lubricants, is not excluded.

Alternatively, in the agent for treating or preventing amyotrophic lateral sclerosis according to the embodiment, the active component consists of at least one selected from among cycloserine and physiologically acceptable salts thereof. The cycloserine may be D-cycloserine or L-cycloserine.

Amyotrophic lateral sclerosis (ALS) is a disease associated with TDP-43 aggregation and is classified as TDP-43 proteinopathy.

The IUPAC name for D-cycloserine (CAS number: 68-41-7) is (4R)-4-Amino-1,2-oxazolidin-3-one. The chemical formula of D-cycloserine is $C_3H_6N_2O_2$. The chemical structural formula of D-cycloserine is as follows.

[Chem. 1]

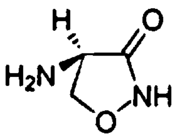

The IUPAC name for L-cycloserine (CAS number: 339-72-0) is (4S)-4-amino-1,2-oxazolidin-3-one. The chemical formula of L-cycloserine is $C_3H_6N_2O_2$. The chemical structural formula of L-cycloserine is as follows.

[Chem. 2]

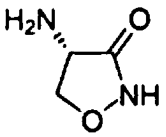

In the agent for treating or preventing amyotrophic lateral sclerosis according to the embodiment, the active component may essentially consist of at least one prodrug of the compound or physiologically acceptable salts thereof. Alternatively, in the agent for treating or preventing amyotrophic lateral sclerosis according to the embodiment, the active component may consist of at least one prodrug of the compound or physiologically acceptable salts thereof.

For example, terizidone is known as a prodrug of D-cycloserine. The IUPAC name for terizidone (CAS number: 25683-71-0) is 4,4'-{1,4-Phenylenebis[(E) methylylidene(E)azanylylidene]}bis(1,2-oxazolidin-3-one). The chemical formula of terizidone is $C_{14}H_{14}N_4O_4$. The chemical structural formula of terizidone is as follows. Terizidone is decomposed in the body and exhibits effects similar to D-cycloserine.

[Chem. 3]

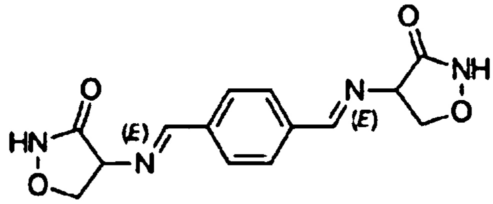

The agent for treating or preventing amyotrophic lateral sclerosis according to the embodiment can be formulated into a pharmaceutically acceptable dosage form. For example, the agent for treating or preventing amyotrophic lateral sclerosis can be formulated into an injectable agent, a solution, a suspension, a tablet, a capsule, a pill, a granule, a syrup, a suppository, an inhalant, or a spray. Examples of injectable agents include an injectable solution, a sterile powder for injection, and a concentrated solution for injection. In the agent for treating or preventing amyotrophic lateral sclerosis according to the embodiment, the content of the active component may be, for example, 15 mg or more and 100 mg or less, 20 mg or more and 80 mg or less, or 25 mg or more and 60 mg or less. In the agent for treating or preventing amyotrophic lateral sclerosis according to the embodiment, the content of D-cycloserine may be, for example, 15 mg or more and 100 mg or less, 20 mg or more and 80 mg or less, or 25 mg or more and 60 mg or less.

It has been reported that, when 15 mg of D-cycloserine is orally administered to humans, the maximum blood concentration (Cmax) of D-cycloserine is about 5.6 μmol/L (van Berckel, B., Lipsch, C., Timp, S. et al. Behavioral and Neuroendocrine Effects of the Partial NMDA Agonist D-cycloserine in Healthy Subjects. Neuropsychopharmacol 16, 317-324

(1997). https://doi.org/10.1016/S0893-133X(96)00196-0, and van Berckel, B. Erratum: Behavioral and Neuroendocrine Effects of the Partial NMDA Agonist D-cycloserine in Healthy Subjects. Neuropsychopharmacol 17, 116 (1997). https://doi.org/10.1016/S0893-133X(97)00082-1).

The brain penetration rate of D-cycloserine has been reported to be 95%. Therefore, the maximum concentration of D-cycloserine in the brain is estimated to be about 5.3 μmol/L. The concentration of about 5.3 μmol/L exceeds an $EC_{50}$ of 128 nmol/L of D-cycloserine, which inhibits cell death in nerve cells forcibly expressing TDP-43, as shown in Example 1 to be described below. In addition, the half-life of D-cycloserine has been reported to be about 10 hours. Therefore, it is estimated that the trough value when 15 mg of D-cycloserine is orally administered twice daily exceeds an $EC_{50}$ of 128 nmol/L. Therefore, when the agent for treating or preventing amyotrophic lateral sclerosis according to the embodiment contains 15 mg or more of D-cycloserine, it is possible to sufficiently exhibit the effect of treating or preventing human amyotrophic lateral sclerosis.

In addition, it has been reported that, when 250 mg of D-cycloserine is administered to humans in order to treat tuberculosis, it causes serious side effects such as epileptiform seizure and mental confusion. In addition, it has been reported that, when 150 mg of D-cycloserine is administered to humans, it causes headaches. Therefore, when the agent for treating or preventing amyotrophic lateral sclerosis according to the embodiment contains 100 mg or less of D-cycloserine, it is possible to inhibit side effects caused by D-cycloserine.

Please note that, in the agent for treating or preventing amyotrophic lateral sclerosis according to the embodiment, the active component may not be in a cationic form. In addition, the active component may not be combined with a compound in an anionic form. The chemical structural formula of the compound in an anionic form is, for example, shown below.

[Chem. 4]

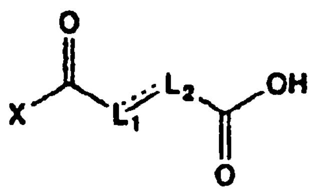

In the chemical structural formula of the compound in an anionic form, X is $-NH_2$ or - OH, L1 and L2 are each independently a $C_{1-6}$ alkylene group, a $C_{2-6}$ alkenylene group, or a $C_{2-6}$ alkynylene group, or as valence allows, one of L1 and L2 is N, O, or S, and the other is a $C_{1-6}$ alkylene group, a $C_{2-6}$ alkenylene group, or a $C_{2-6}$ alkynylene group. The compound in an anionic form is, for example, succinic acid, D-tartaric acid, L-tartaric acid, mesotartaric acid, fumaric acid, maleic acid, or malic acid. The combination of an active component in a cationic form and a compound in an anionic form may cause the active component to become free, thereby decreasing its solubility in water, and leading to a deterioration in the quality of the active component.

In addition, in the agent for treating or preventing amyotrophic lateral sclerosis according to the embodiment, the active component may not be combined with acamprosate. Acamprosate is a derivative of homotaurine, and also known as 3-(acetylamino)propylsulfonic acid or N-acetylhomotaurine. When acamprosate is administered to humans, it can cause side effects such as anaphylaxis associated with symptoms such as generalized skin rash, rash, urticaria, stomatitis, laryngospasm, and shortness of breath. In addition, when acamprosate is administered to humans, it can cause side effects such as angioedema associated with symptoms such as tongue swelling and lymphadenopathy. Since the agent for treating or preventing amyotrophic lateral sclerosis according to the embodiment does not need to be combined with acamprosate, these side effects do not occur.

The agent for treating or preventing amyotrophic lateral sclerosis according to the embodiment can treat or prevent amyotrophic lateral sclerosis by inhibiting TDP-43 aggregation. Alternatively, the agent for treating or preventing amyotrophic lateral sclerosis according to the embodiment can treat or prevent amyotrophic lateral sclerosis by inhibiting cell death in TDP-43-overexpressing cells.

While the present invention has been described above with reference to the embodiments, the descriptions and drawings that form some of the disclosure should not be understood as limiting the invention. Those skilled in the art can clearly understand various alternative embodiments, examples and operational techniques from this disclosure. It should be understood that the present invention includes various embodiments and the like that are not described herein.

(Reference Example 1)

Mouse neuroblastoma (Neuro2a) cells were seeded on a dish. The Neuro2a cells were cultured in a culture medium containing DMEM+10% FBS at 37°C in the presence of 5% $CO_2$. On the first day after seeding, differentiation into neurons was initiated. Differentiation into neurons was performed in a culture medium containing DMEM+2% FBS+20 μmol/L retinoic acid. On the fourth day after seeding, cells were transfected with synthesized TDP-43 mRNA by a lipofection method. Cells were sampled at 6 hours, 9 hours, 12 hours, 15 hours, 18 hours, and 21 hours after transfection. At 21 hours after transfection, cell death was observed. For sampling, cells were lysed in an RIPA buffer and centrifuged at 22,000 Xg for 30 minutes at 4°C, the supernatant was used as the soluble fraction, the precipitate was used as the insoluble fraction, and equal volumes of a 2×SDS sample buffer were added to the respective fractions and heated at 95°C for 5 minutes to prepare electrophoresis samples. The electrophoresis samples were subjected to electrophoresis using a polyacrylamide gel, TDP-43 was then transferred to a PVDF membrane. TDP-43 was detected through chemiluminescence using anti-TDP-43 antibodies as primary antibodies and HRP-conjugated anti-rabbit antibodies as secondary antibodies. These results are shown in Figure 1 and Figure 2. It was confirmed that the number of insoluble fractions of TDP-43 increased in the cells between the transfection of the TDP-43 gene and the onset of cell death. The insoluble fractions of TDP-43 indicate the aggregation of TDP-43.

(Example 1)

Neuro2a cells were seeded on a dish. On the first day after seeding, differentiation into neurons was initiated. On the fourth day after seeding, cells were transfected with the TDP-43 gene. At 5 hours after transfection, 10 nmol/L, 50 nmol/L, 100 nmol/L, 500 nmol/L, 1,000 nmol/L, 5,000 nmol/L, or 10,000 nmol/L of D-cycloserine or L-cycloserine was added to a culture medium. In addition, as a control, 10 nmol/L, 50 nmol/L, 100 nmol/L, 500 nmol/L, 1,000 nmol/L, 5,000 nmol/L, or 10,000 nmol/L of ropinirole was added to a culture medium. Ropinirole has been reported to have an effect of treating ALS.

The proportion of dead cells was evaluated 7 days after the cells were transfected with the TDP-43 gene. As shown in Figure 3A, no cell death was observed in cells which were not transfected with the TDP-43 gene. As shown in Figure 3B, cell death was observed in cells which were transfected with the TDP-43 gene and not supplemented with D-cycloserine or edaravone. As shown in Figure 3C, cell death was inhibited in cells which were transfected with the TDP-43 gene and supplemented with D-cycloserine.

As shown in Figure 4, D-cycloserine and L-cycloserine exhibited an effect of inhibiting cell death in a concentration-dependent manner. The $EC_{50}$ of D-cycloserine was 128 nmol/L. The $EC_{50}$ of L-cycloserine was 237 nmol/L. The $EC_{50}$ of ropinirole was 198 nmol/L. Here, the relative viability indicates the proportion of the number of viable cells overexpressing TDP-43 based on 100% of the number of viable cells not overexpressing TDP-43.

(Example 2)

Neuro2a cells were seeded on a dish. On the first day after seeding, differentiation into neurons was initiated. On the fourth day after seeding, cells were transfected with the TDP-43 gene. At 6 hours after transfection, 10 μmol/L of D-cycloserine, L-cycloserine, ropinirole, or DMSO was added to the cells, and the cells were cultured for 12 hours. Then, the results obtained by analyzing the soluble fractions and insoluble fractions of TDP-43 in the cells are shown in Figure 5. As shown in Figure 5, as a control, in cells treated with DMSO, it was confirmed that the number of insoluble fractions of TDP-43 was larger than the number of soluble fractions of TDP-43. On the other hand, in cells treated with D-cycloserine and L-cycloserine, it was confirmed that the number of soluble fractions of TDP-43 was larger than the number of insoluble fractions of TDP-43.

The results obtained by quantifying the proportion of insoluble fractions based on a total number of soluble fractions and insoluble fractions of TDP-43 in the cells are shown in Figure 6. In cells treated with D-cycloserine and L-cycloserine, the proportion of insoluble fractions was less than half.

(Example 3)

100 μL of an EHS-gel basement membrane matrix (commercially available from FUJIFILM Wako Pure Chemical Corporation) was added to 12.5 mL of DMEM (Dulbecco's Modified Eagle Medium, commercially available from FUJIFILM Wako Pure Chemical Corporation) and mixed, and the mixture was added to a 24-well plate at 600 μL per well. The plate was left at room temperature for 2 hours, and the solution was then removed from the wells. Next, 600 μL of DMEM was added to each well, and the plate was left at room temperature for 2 hours.

Motor neurons derived from iPS cells of healthy subjects and motor neurons derived from iPS cells of ALS patients were obtained from iXCells Biotechnologies. The motor neurons were quickly thawed in a thermostatic oven at 37°C and suspended in a motor neuron maintenance culture medium (iXCells Biotechnologies). The culture medium was centrifuged at 600 Xg for 5 minutes to collect motor neurons, the culture medium was then removed, and the motor neurons were suspended in a motor neuron maintenance culture medium.

After motor neurons were diluted in a culture medium to a density of $3X10^5$ cells/mL, 600 μL of the culture medium containing motor neurons was added to each of a plurality of wells of a matrix-coated plate, and the motor neurons were cultured at 37°C in the presence of 5% $CO^2$. Every 2 or 3 days, 300 μL of the culture medium was removed from each of the plurality of wells, and 300 μL of a new culture medium was added to each of the plurality of wells.

On the seventh day after culture initiation, 300 μL of the culture medium was removed from each of the plurality of wells, and 300 μL of a culture medium containing 0.2% DMSO, a culture medium containing 20 μmol/L of D-cycloserine (Cayman Chemical) or a culture medium containing 20 μmol/L of ropinirole (commercially available from FUJIFILM Wako Pure Chemical Corporation) was added to each of the plurality of wells. Every 2 or 3 days, 300 μL of the culture medium was removed from each of the plurality of wells, 300 μL of one containing the same compound as the removed culture medium, that is, a culture medium containing 0.1% DMSO, a culture medium containing 10 μmol/L of D-cycloserine, or a culture medium containing 10 μmol/L of ropinirole, was added to each of the plurality of wells.

5 μL of a protease inhibitor and phosphatase inhibitor cocktail (Halt Protease and Phosphatase Inhibitor Cocktail, 10OX, registered trademark, Thermo Fisher Scientific) was added to 500 μL of a protein extraction buffer solution containing a surfactant (RIPA buffer, commercially available from Nacalai Tesque, Inc.) to prepare a cell extraction solution. 50 μL of the cell extraction solution was added to the motor neurons cultured for 20 days in the presence of DMSO, D-cycloserine, or ropinirole, and the cell extraction solution was collected in a 1.5 mL tube by pipetting. The cell extraction solution

was centrifuged at 4°C, 21,000 Xg for 30 minutes.

After centrifugation, 45 μL of the supernatant was collected, 45 μL of an electrophoresis buffer solution (AE-1430 EzApply, Atto) was then added to the supernatant and heated at 95°C for 5 minutes to prepare soluble protein fractions. In addition, after centrifugation, the precipitate was washed twice with 50 μL of an RIPA buffer, 45 μL of the RIPA buffer and 45 μL of the electrophoresis buffer solution were then added to the precipitate and heated at 95°C for 5 minutes to prepare insoluble protein fractions.

The soluble protein fractions and the insoluble protein fractions were subjected to SDS-PAGE, and the proteins were transferred to a PVDF membrane. The PVDF membrane was immersed in a blocking solution and shaken at room temperature for 1 hour to block the PVDF membrane. The PVDF membrane was immersed in solution containing TDP-43 polyclonal antibodies (PGI Proteintech Group) and shaken at 4°C overnight. Then, the PVDF membrane was washed three times with a buffer solution, and the PVDF membrane was then immersed in a solution containing HRP (horseradish peroxidase)-labeled secondary antibodies (Anti-Rabbit IgG, HRP-Linked F(ab')2 Fragment Donkey, Cytiva) and shaken at room temperature for 1 hour. Next, the PVDF membrane was washed three times with a buffer solution, 1 mL of a luminescence reagent (ImmunoStar LD, commercially available from FUJIFILM Wako Pure Chemical Corporation) was then used to induce luminescence from the labeled secondary antibodies, and the PVDF membrane was imaged. The imaged PVDF membrane is shown in Figure 7.

As shown in Figure 7, as a control, in motor neurons derived from iPS cells of healthy subjects cultured in the presence of DMSO, bands for the soluble fractions of TDP-43 were clearly observed, and bands for insoluble fractions were unclear. In motor neurons derived from iPS cells of healthy subjects cultured in the presence of D-cycloserine, bands for soluble fractions of TDP-43 were clearly observed, and bands for insoluble fractions were unclear. In motor neurons derived from iPS cells of healthy subjects cultured in the presence of ropinirole, bands for soluble fractions of TDP-43 were clearly observed, and bands for insoluble fractions were unclear.

As shown in Figure 7, as a control, in motor neurons derived from iPS cells of ALS patients cultured in the presence of DMSO, bands for soluble fractions of TDP-43 were clearly observed, and bands for insoluble fractions were also clearly confirmed. In motor neurons derived from iPS cells of ALS patients cultured in the presence of D-cycloserine, bands for soluble fractions of TDP-43 were clearly observed, and bands for insoluble fractions were unclear. In motor neurons derived from iPS cells of ALS patients cultured in the presence of ropinirole, bands for soluble fractions of TDP-43 were clearly observed, and bands for insoluble fractions were unclear.

The captured PVDF membrane image was opened in ImageJ software and the Invert command was used to invert black and white. After the background was removed using the Subtract Background command, the TDP-43 bands in the lanes were sequentially enclosed in rectangular frames of the same size, and the band intensity was quantified using the Measure command. The proportion of insoluble fractions of TDP-43 was calculated from the band intensity of soluble fractions of TDP-43 and the band intensity of insoluble fractions of TDP-43 for each sample according to the following formula.

$$R=\{I_I/(I_I+I_S)\}\times 100$$

In the formula, R is the proportion (%) of insoluble fractions of TDP-43, $I_I$ is the band intensity of insoluble fractions of TDP-43, and $I_S$ is the band intensity of soluble fractions of TDP-43.

Three experiments were performed, and the average proportion of insoluble fractions of TDP-43 was calculated. In Figure 8, the proportion of insoluble fractions of TDP-43 in each experiment is shown as a dot, and the average value is shown as a bar. In motor neurons derived from iPS cells of healthy subjects, the average proportion of insoluble fractions of TDP-43 was about 7.4%. On the other hand, in motor neurons derived from iPS cells of ALS patients, the average proportion of insoluble fractions of TDP-43 was about 46.1%. However, in motor neurons derived from iPS cells of ALS patients in the presence of D-cycloserine, the average proportion of insoluble fractions of TDP-43 decreased to about 17.4%. In addition, in motor neurons derived from iPS cells of ALS patients in the presence of ropinirole, the average proportion of insoluble fractions of TDP-43 decreased to about 19.3%.

## Claims

1. An agent for treating or preventing amyotrophic lateral sclerosis, wherein an active component of the agent essentially consists of at least one selected from the group consisting of cycloserine, terizidone, and salts thereof.

2. The agent for treating or preventing amyotrophic lateral sclerosis according to claim 1, wherein the active component consists of at least one selected from the group consisting of cycloserine, terizidone, and salts thereof.

3. The agent for treating or preventing amyotrophic lateral sclerosis according to claim 1, wherein the active component essentially consists of cycloserine or salt thereof.

**4.** The agent for treating or preventing amyotrophic lateral sclerosis according to claim 1, wherein the active component consists of cycloserine or salt thereof.

**5.** The agent for treating or preventing amyotrophic lateral sclerosis according to claim 1, wherein the cycloserine is D-cycloserine.

**6.** The agent for treating or preventing amyotrophic lateral sclerosis according to claim 1, wherein the cycloserine is L-cycloserine.

**7.** The agent for treating or preventing amyotrophic lateral sclerosis according to claim 1, containing 15 mg or more and 100 mg or less of the active component.

**8.** The agent for treating or preventing amyotrophic lateral sclerosis according to claim 1, containing 15 mg or more and 100 mg or less of D-cycloserine.

**9.** The agent for treating or preventing amyotrophic lateral sclerosis according to claim 1, wherein the active component is not in a cationic form.

**10.** The agent for treating or preventing amyotrophic lateral sclerosis according to claim 1, wherein the active component is not combined with a compound in an anionic form.

**11.** The agent for treating or preventing amyotrophic lateral sclerosis according to claim 1, wherein the active component is not combined with acamprosate.

Fig. 1

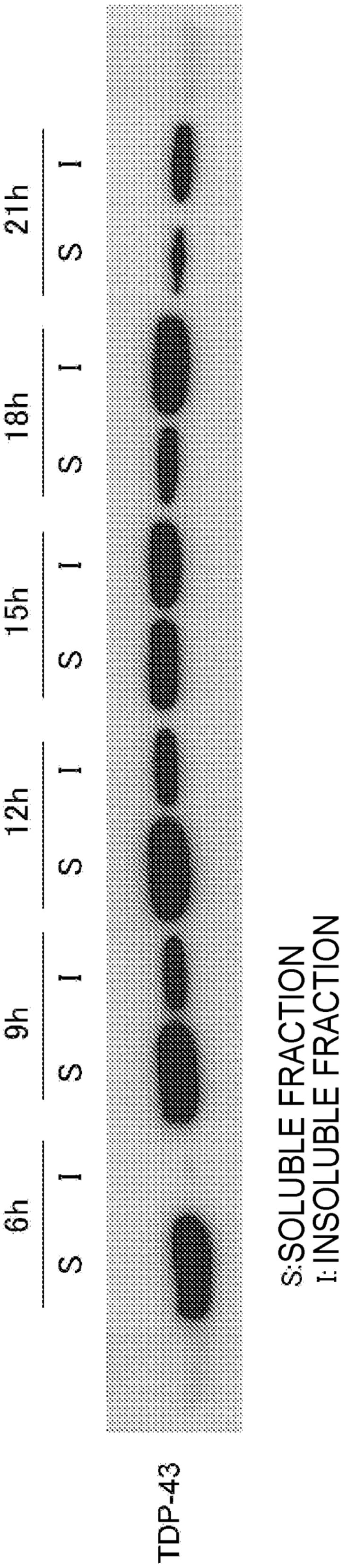
6h
9h
12h
15h
18h
21h
S I S I S I S I S I S I
TDP-43
S:SOLUBLE FRACTION
I: INSOLUBLE FRACTION

Fig. 2

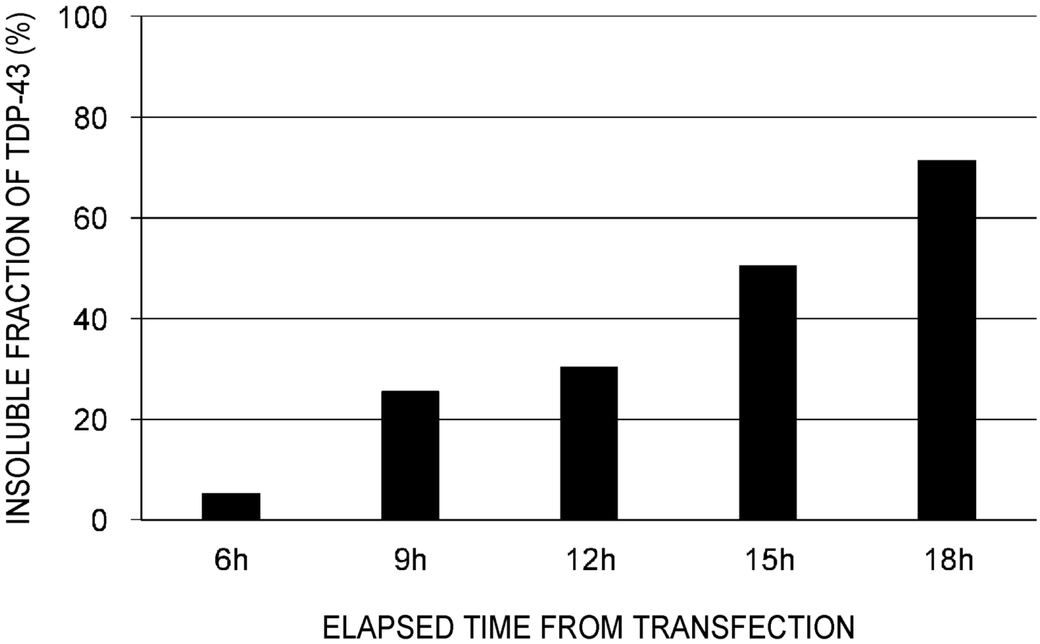
INSOLUBLE FRACTION OF TDP-43 (%)
100
80
60
40
20
0
6h
9h
12h
15h
18h
ELAPSED TIME FROM TRANSFECTION

Fig. 3

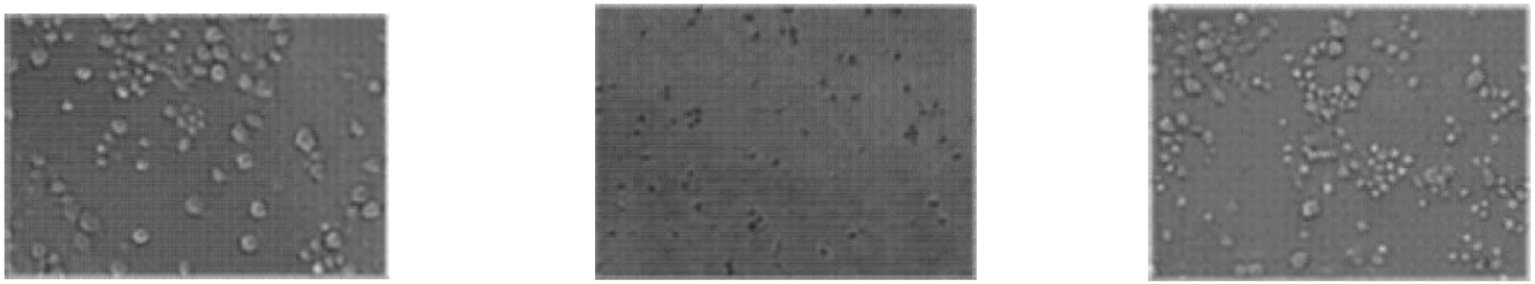

A WITHOUT TRANSFECTION

B WITH TRANSFECTION

C WITH TRANSFECTION, ADD 10 μmol/L OF D-cycloserine

Fig. 4

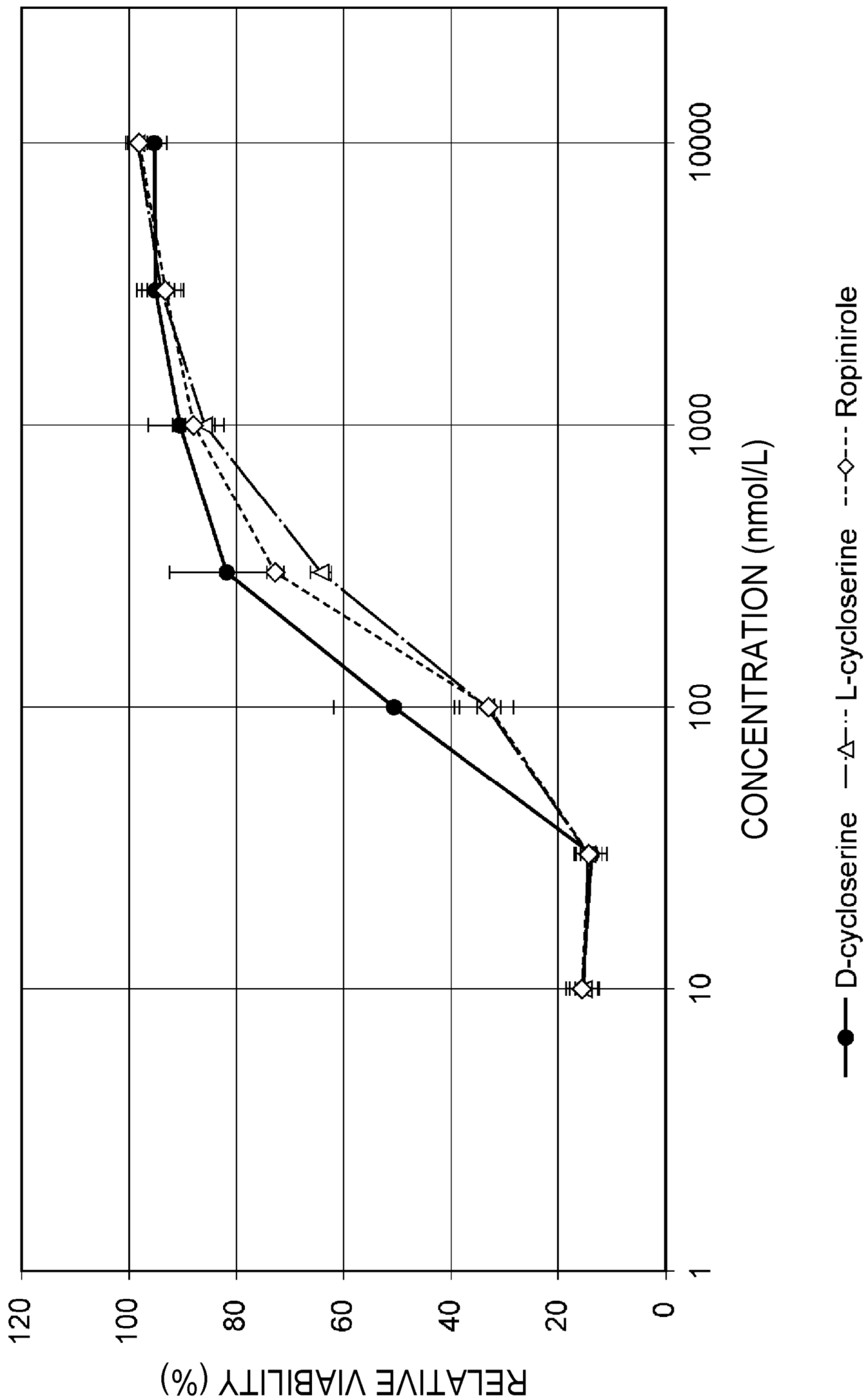
RELATIVE VIABILITY (%)
120
100
80
60
40
20
0
CONCENTRATION (nmol/L)
1
10
100
1000
10000
D-cycloserine
L-cycloserine
Ropinirole

Fig. 5

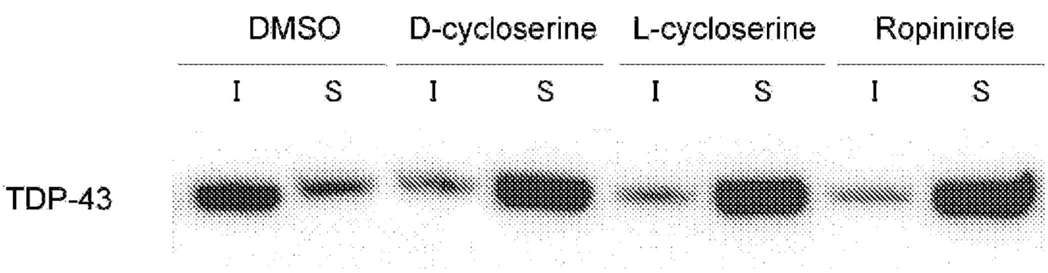


S:SOLUBLE FRACTION
I: INSOLUBLE FRACTION

Fig. 6

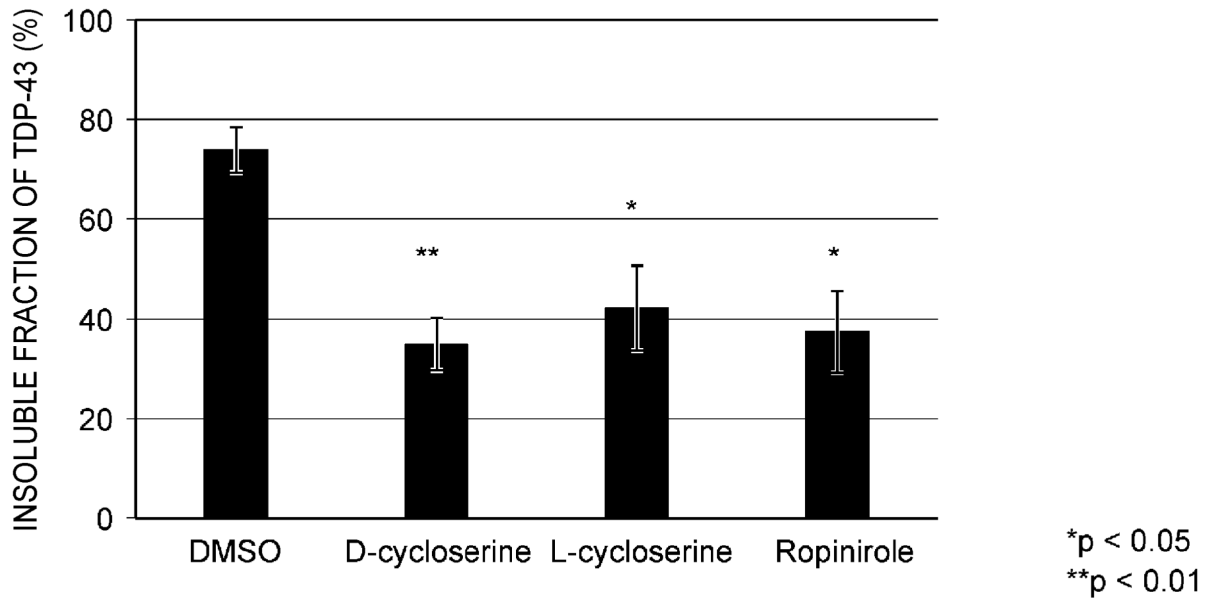


| | DMSO | D-cycloserine | L-cycloserine | Ropinirole |
|---|---|---|---|---|
| INSOLUBLE TDP-43 (%) | 73.87 | 34.93 | 42.19 | 37.42 |

Fig. 7

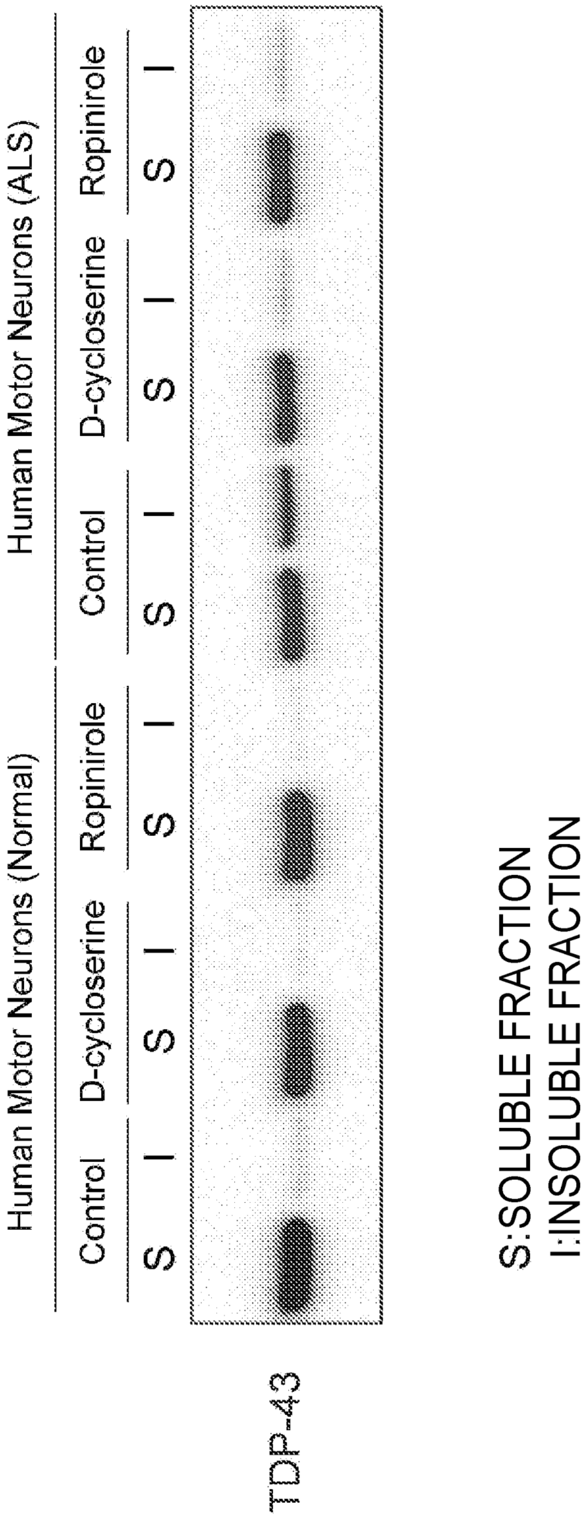
Human Motor Neurons (Normal)
Control
D-cycloserine
Ropinirole
S
I
S
I
S
I
Human Motor Neurons (ALS)
Control
D-cycloserine
Ropinirole
S
I
S
I
S
I
TDP-43
S:SOLUBLE FRACTION
I:INSOLUBLE FRACTION

Fig. 8

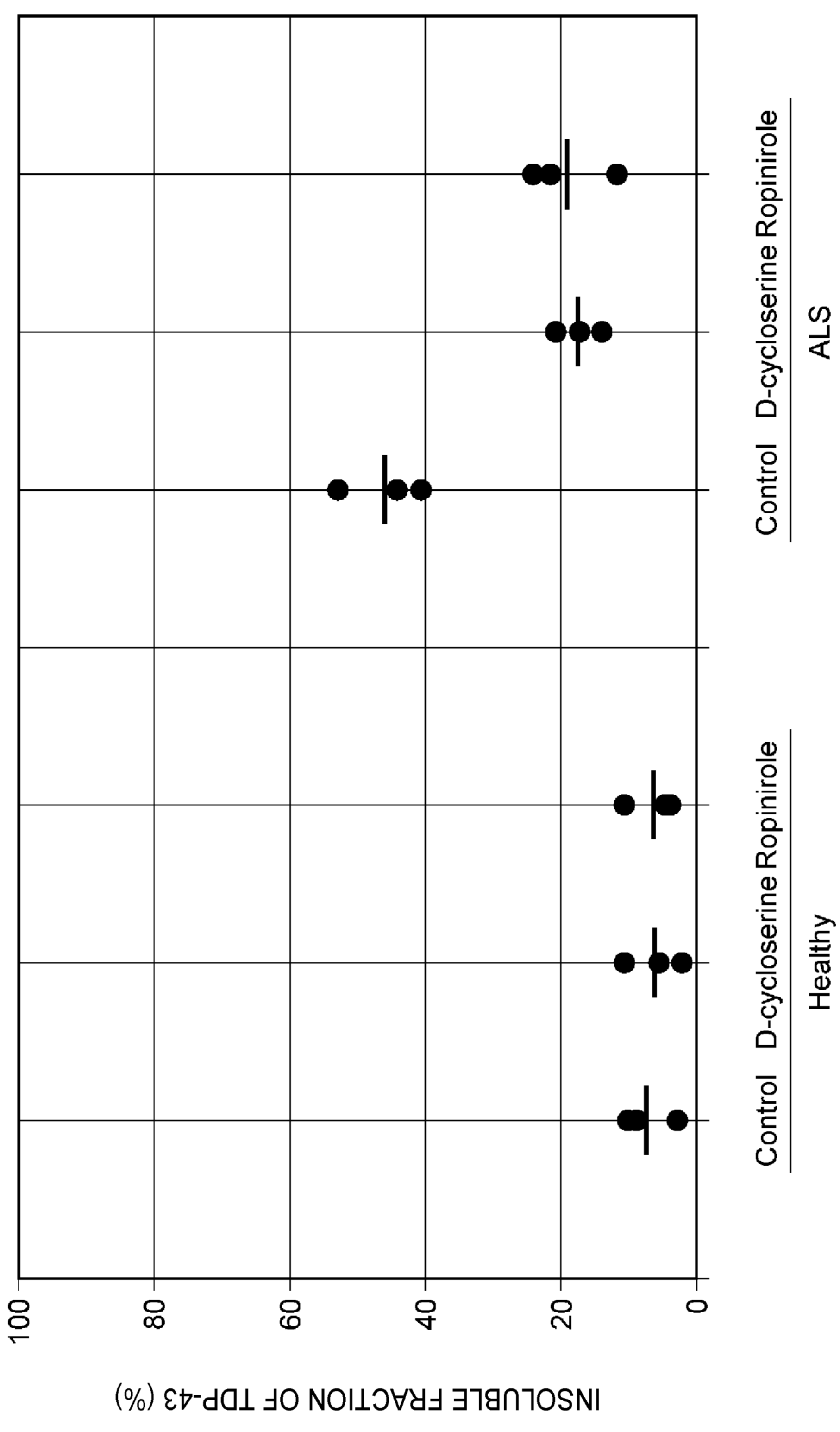
INSOLUBLE FRACTION OF TDP-43 (%)
100
80
60
40
20
0
Control
D-cycloserine
Ropinirole
Healthy
Control
D-cycloserine
Ropinirole
ALS

**INTERNATIONAL SEARCH REPORT**

International application No. **PCT/JP2023/016569**

**A. CLASSIFICATION OF SUBJECT MATTER**

***A61K 31/42***(2006.01)i; ***A61P 25/28***(2006.01)i
FI: A61K31/42; A61P25/28

According to International Patent Classification (IPC) or to both national classification and IPC

**B. FIELDS SEARCHED**

Minimum documentation searched (classification system followed by classification symbols)

A61K31/42; A61P25/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2022-500403 A (SYNEURX INTERNATIONAL (TAIWAN) CORP.) 04 January 2022 (2022-01-04)<br>paragraph [0003], claims | 1-11 |
| Y | JP 2022-500397 A (SYNEURX INTERNATIONAL (TAIWAN) CORP.) 04 January 2022 (2022-01-04)<br>paragraphs [0003], [0163], claims | 1-11 |
| Y | PAUL, Praveen et al. The role of D-serine and glycine as co-agonists of NMDA receptors in motor neuron degeneration and amyotrophic lateral sclerosis (ALS). Frontiersin Synaptic Neuroscience. 2014, vol. 6, article 10, pp. 1-6<br>abstract, fig. 1-2 | 1-11 |
| Y | SASABE, Jumpei et al. D-Serine is a key determinant of glutamate toxicity in amyotrophic lateral sclerosis. The EMBO Journal. 2007, vol. 26, pp. 4149-4159<br>abstract, fig. 3, p. 4151, left column, lines 18-25 | 1-11 |
| X | JP 2021-152060 A (CONFLUENCE PHARMACEUTICALS, LLC) 30 September 2021 (2021-09-30)<br>paragraphs [0002]-[0005], [0059]-[0063], claims | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 July 2023** | **01 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
PCT/JP2023/016569

C. DOCUMENTS CONSIDERED TO BE RELEVANT

| Category* | Citation of document, with indication, where appropriate, of the relevant passages | Relevant to claim No. |
|---|---|---|
| A | 笹部 潤平, D-セリン代謝異常と筋萎縮性側索硬化症, D-アミノ酸研究会誌, 2013, vol.1 (1), pp. 7-11, non-official translation (SASABE, Jumpei. D-serine metabolic disorders and amyotrophic lateral sclerosis. journal of D-amino acid research.) p. 8, left column, lines 19-30 | 1-11 |
| A | ARSENI, Diana et al. Structure of pathological TDP-43 filaments from ALS with FTLD. Nature. 2022, vol. 601, pp. 139-143 whole document | 1-11 |
| A | EGAWA, Naohiro et al. TDP-43 regulates cholesterol biosynthesis by inhibiting sterol regulatory element-binding protein 2. Scientific Reports. 2022, vol. 12:7988, pp. 1-12 whole document | 1-11 |
| A | JP 2014-505095 A (WISTA LABORATORIES LTD.) 27 February 2014 (2014-02-27) whole document | 1-11 |

Form PCT/ISA/210 (second sheet) (January 2015)

**INTERNATIONAL SEARCH REPORT**
**Information on patent family members**

International application No.
**PCT/JP2023/016569**

| Patent document cited in search report | Publication date (day/month/year) | Patent family member(s) | Publication date (day/month/year) |
|---|---|---|---|
| JP 2022-500403 A | 04 January 2022 | US 2020/0085792 A1<br>paragraph [0002], claims<br>EP 3849527 A1<br>KR 10-2021-0060478 A<br>CN 113382722 A | |
| JP 2022-500397 A | 04 January 2022 | US 2022/0047561 A1<br>paragraphs [0003], [0172], claims<br>EP 3849547 A1<br>CN 112714645 A<br>KR 10-2021-0061348 A | |
| JP 2021-152060 A | 30 September 2021 | US 2018/0221315 A1<br>paragraphs [0002]-[0005], [0060]-[0064], claims<br>EP 3331518 A1<br>KR 10-2018-0034442 A | |
| JP 2014-505095 A | 27 February 2014 | US 2013/0315992 A1<br>whole document<br>EP 2673266 A1<br>KR 10-2014-0007428 A<br>CN 103649061 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5898701 B
- JP 2022500397 W
- JP 2018526345 W
- US 20110160260
- JP 2002511409 W


### Non-patent literature cited in the description


- *CHEMICAL ABSTRACTS*, 68-41-7
- *CHEMICAL ABSTRACTS*, 339-72-0
- *CHEMICAL ABSTRACTS*, 25683-71-0
- **VAN BERCKEL, B.** ; **LIPSCH, C.** ; **TIMP, S. et al.** Behavioral and Neuroendocrine Effects of the Partial NMDA Agonist D-cycloserine in Healthy Subjects. *Neuropsychopharmacol*, 1997, vol. 16, 317-324, https://doi.org/10.1016/S0893-133X(96)00196-0
- **VAN BERCKEL, B.** Behavioral and Neuroendocrine Effects of the Partial NMDA Agonist D-cycloserine in Healthy Subjects. *Neuropsychopharmacol*, 1997, vol. 17, 116, https://doi.org/10.1016/S0893-133X(97)00082-1